(19) Europäisches Patentamt
European Patent Office
Office européen des brevets

(11) Publication number: 0 003 622
B1

(12) EUROPEAN PATENT SPECIFICATION

(45) Date of publication of patent specification: 13.01.82

(21) Application number: 79200047.3

(22) Date of filing: 24.01.79

(51) Int. Cl.³: C 07 C 15/08, C 07 C 7/00, C 07 C 7/13, C 07 C 7/14, C 07 C 5/27, B 01 J 29/04 //C01B33/20

# (54) Process for the preparation and separation of para-xylene.

(30) Priority: 07.02.78 NL 7801367

(43) Date of publication of application: 22.08.79 Bulletin 79/17

(45) Publication of the grant of the European patent: 13.01.82 Bulletin 82/2

(84) Designated Contracting States: BE CH DE FR GB IT

(56) References cited:
DE - A - 2 755 770
FR - A - 2 142 999
GB - A - 1 420 796

(73) Proprietor: SHELL INTERNATIONALE RESEARCH MAATSCHAPPIJ B.V.
Carel van Bylandtlaan 30
NL-2596 HR DEN HAAG (NL)

(72) Inventor: Maas, Rudolf Jakob
Badhuisweg 3
NL-1031 CM Amsterdam (NL)
Inventor: Visser, René Maria
Badhuisweg 3
NL-1031 CM Amsterdam (NL)

(74) Representative: Keuzenkamp, Abraham et al,
P.O. Box 302
NL-2501 CH Den Haag (NL)

EP 0 003 622 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Process for the preparation and separation of para-xylene

The invention relates to a process for the preparation and separation of para-xylene from a mixture substantially consisting of aromatic hydrocarbons with eight carbon atoms in the molecule.

Para-xylene is an important base material for the chemical industry, which is used on a large scale for the preparation of terephthalic acid. As a rule, the preparation starts from mixtures of aromatic hydrocarbons with eight carbon atoms in the molecule, which mixtures are obtained by applying a combination of distillation and selective extraction to hydrocarbon streams obtained in processing mineral oil or coal. In these mixtures para-xylene is usually present together with the isomeric compounds ortho-xylene, meta-xylene and ethylbenzene. Since the four isomeric compounds resemble one another closely as regards physical and chemical properties, the separation of pure para-xylene from these mixtures and from mixtures whose para-xylene content has been increased by isomerization, presents a problem. In the past many methods have been proposed for the preparation and separation of para-xylene from mixtures of aromatic hydrocarbons with eight carbon atoms in the molecule, but as a rule a para-xylene so obtained is contaminated to a greater or less degree with one or more of the isomers.

In an investigation by the Applicant concerning this subject it has been found that certain crystalline silicates which have been synthesized recently for the first time by the Applicant, are pre-eminently suitable for use in a process for the preparation and separation of para-xylene from the above-mentioned mixtures. In fact it has been found that these crystalline silicates are capable not only of adsorbing very selectively para-xylene and ethylbenzene from mixtures of the four isomers such as can be separated, for instance, in the processing of mineral oil or coal, but also of adsorbing very selectively para-xylene from mixtures of the three xylenes obtained in the isomerization of a mixture of ortho-xylene and meta-xylene. The new crystalline silicates having these extraordinary properties are characterized as follows:

(a) they are thermally stable up to temperatures higher than 600°C,

(b) they are, after dehydration at 400°C in vacuum, capable of adsorbing more than 3 %w water at 25°C and saturated water vapour pressure, and

(c) in dehydrated form, they have the following overall composition, expressed in moles of the oxides:

$(1.0\pm0.3)(R)_{2/n}O.\ [a\ Fe_2O_3.b\ Al_2O_3.c\ Ga_2O_3].\ y(d\ SiO_2.e\ GeO_2)$, where
R=one or more mono- or bivalent, cations,
$a\geqslant0.1$,
$b\geqslant0$,
$c\geqslant0$,
$a+b+c=1$,
$y\geqslant10$,
$d\geqslant0.1$,
$e\geqslant0$,
$d+e=1$, and
n=the valency of R.

By using the above-mentioned extraordinary properties of the crystalline silicates in two separate adsorption steps and combining these adsorption steps with an isomerization step applied to a mixture of ortho-xylene and meta-xylene and a crystallization step applied to a mixture of para-xylene and ethylbenzene, the Applicant has succeeded in developing a process which can be used for preparing para-xylene in high yield and separating it in a pure form from mixtures substantially consisting of aromatic hydrocarbons with eight carbon atoms in the molecule.

The present patent application therefore relates to a process for the preparation and separation of para-xylene from a mixture substantially consisting of aromatic hydrocarbons with eight carbon atoms in the molecule, in which the mixture is separated by adsorption at elevated temperature into a mixture of para-xylene and ethylbenzene and a mixture of ortho-xylene and meta-xylene, the para-xylene is separated by crystallization after cooling of the mixture of para-xylene and ethylbenzene, a resulting mixture of ortho-xylene and meta-xylene is isomerized with a catalyst at elevated temperature and a pressure from 1 up to 150 bar, a further quantity of para-xylene is separated from the isomerized product by adsorption at elevated temperature and use is made in both adsorption treatments of a crystalline silicate as adsorbent, characterized in that the crystalline silicate is as defined hereinbefore.

In the British patent specification 1,420,796 a process for the preparation and separation of para-xylene, as described above, but with ZSM 5 and ZSM 8 zeolites as adsorbents is disclosed. The mixture of para-xylene and ethylbenzene after desorption with steam still contains 9 percent by weight of meta-xylene on the average.

The process according to the invention is primarily intended for the preparation and separation of para-xylene from mixtures of $C_8$ aromatics. However, the process presents the additional possibility of separating a part of the ortho-xylene and/or meta-xylene present in these mixtures and practically all of the ethylbenzene present therein in a pure form. For the separation of part of the ortho-xylene and/or

meta-xylene from these mixtures, the non-adsorbed mixture of ortho-xylene and meta-xylene from the first adsorption step is subjected to fractional distillation, in which the lower-boiling meta-xylene distils over first. In addition to the possibility of separating part of the ortho-xylene and/or meta-xylene present in the starting mixture in a pure form by incorporating a distillation step between the first adsorption step and the isomerization step, the incorporation of this distillation step is also important for two other reasons. The first reason relates to the composition of the mixtures of $C_8$ aromatics which are used as the feed in the process according to the invention. Besides the four isomeric $C_8$ aromatics, these mixtures often also contain small quantities of aromatic hydrocarbons with 9 and more carbon atoms in the molecule. Like ortho-xylene and meta-xylene, these $C_9^+$ aromatics are not adsorbed by the crystalline silicate and consequently find their way into the feed for the isomerization step. Incorporation of a distillation step after the first adsorption step has the advantage that these $C_9^+$ aromatics are left as a bottom product in the distillation. The second reason why the inclusion of a distillation step before the isomerization step is important, relates to a special embodiment of the process according to the invention, in which non-adsorbed product from the second adsorption step is recirculated to the isomerization step in order to increase the yield of para-xylene. Owing to the occurrence of side reactions the product of the isomerization step often contains, in addition to the three isomeric xylenes, small quantities of other hydrocarbons as well, in particular $C_9$ aromatics. Since these by-products of the isomerization, just like ortho-xylene and meta-xylene, are not as a rule adsorbed by the crystalline silicate, they find their way into the recirculation stream to the isomerisation step. Incorporation of a distillation step before the isomerization step has the advantage that these by-products can be removed from the recirculation stream by passing the recirculation stream through this distillation step. As was mentioned hereinbefore, the process according to the invention, in addition to allowing the preparation and separation of the para-xylene from mixtures of $C_8$ aromatics, presents the possibility of separating practically all of the ethylbenzene present therein in a pure form. For the separation of ethylbenzene from these mixtures the mother liquor from the crystallization step is subjected to distillation. The ethylbenzene can thereby be distilled off in a pure form. To increase the yield of para-xylene, the distillation residue containing para-xylene is preferably recirculated to the crystallization step in the process according to the invention. It may happen that in the treatment of the mixture of the three xylenes and ethylbenzene in the first adsorption step a small portion of the ortho-xylene and/or meta-xylene is adsorbed as well. During the further processing of the adsorbed product from the first adsorption step, this ortho-xylene and/or meta-xylene will eventually find its way into the distillation residue of the ethyl-benzene recovery. To avoid a build-up of ortho-xylene and/or meta-xylene in the recirculation stream, the distillation residue in question is preferably recirculated to the first adsorption step instead of the crystallization step.

The mixture of ethylbenzene, para-xylene and, optionally, small quantities of ortho-xylene and meta-xylene from the crystallization step may very conveniently be used as the feed for an additional isomerization process for the preparation of para-xylene. The ethylbenzene that can be separated in the process according to the invention, may be used as a gasoline component or as a base material for the chemical industry, for instance for the preparation of styrene.

The process according to the invention comprises two separate adsorption steps with use of a crystalline silicate as the adsorbent. The adsorptions are carried out by contacting the feed at elevated temperature, for instance at a temperature between 25 and 225°C, with the adsorbent. The adsorbed compounds can be isolated from the adsorbent in various ways. Desorption may, for instance, be effected by heating the adsorbent, by reducing the pressure in the space in which the adsorbent is present or by treating the adsorbent with a suitable inert gas or a displacing agent.

The process according to the invention further comprises a crystallization step. The crystallization is carried out by cooling the feed to a temperature which is sufficiently low for the crystallization to take place. Suitable temperatures lie between —15 and —80°C. As a rule, the crystallization is started by seeding.

Finally, the process according to the invention comprises an isomerization step. The isomerization is carried out by contacting the feed at elevated temperature with an isomerization catalyst. The isomerization may be carried out both at atmospheric pressure and at elevated pressures up to about 150 bar. The isomerization is preferably carried out in the liquid phase at a temperature lower than 350°C and in the presence of 5—30%w and in particular of 15—25%w toluene added as diluent. As catalysts that may be used in the isomerization step according to the invention, all catalysts that are capable of effecting the desired conversion of ortho-xylene and meta-xylene into para-xylene are in principle eligible.

In the investigation concerning the preparation and separation of para-xylene from mixtures of $C_8$ aromatics the Applicant has found that the above-mentioned crystalline silicates are not only very suitable for use as adsorbent in the separation of isomeric $C_8$ aromatics, but that these crystalline silicates also show a high activity, selectivity and stability for catalysing the isomerization of ortho-xylene and meta-xylene into para-xylene. In the process according to the invention it is therefore highly preferred to use such a crystalline silicate as catalyst in the isomerization step.

In the process according to the invention it is preferred both for the two adsorption steps and for the isomerization step to use crystalline silicates which contain no gallium or germanium, in other

words silicates of which, in the above-mentioned overall composition, c and e are 0. Such silicates are the subject of Netherlands patent application No. 7613957. Further, preference is given to the use of silicates of which, in the abovementioned overall composition, a is greater than 0.5 and preferably equal to 1. Particular preference is given to silicates which contain no aluminium, in other words silicates of which, in the above-mentioned overall composition, b is 0. It should be noted that in the silicates used in the process according to the invention, y is preferably less than 600 and in particular less than 300. Finally, in the process according to the invention preference is given to silicates whose X-ray powder diffraction pattern has, inter alia, the reflections mentioned in Table A of Netherlands patent application No. 7613957.

The crystalline silicates which, in the process according to the invention, are used as adsorbent and, moreover, preferably as isomerization catalyst, are as a rule prepared from an aqueous mixture as the starting material which comprises the following compounds in a given ratio: one or more compounds of an alkali metal, one or more compounds containing an organic cation or from which such a cation is formed during the preparation of the silicate, one or more silicon compounds, one or more iron compounds and, optionally, one or more aluminium, gallium and/or germanium compounds. The preparation takes place by maintaining the mixture at elevated temperature until the silicate has been formed and subsequently separating the crystals of the silicate from the mother liquor. The silicates prepared in this way contain alkali metal ions and organic cations. Before being used in the process according to the invention, the organic cations which have been introduced during the preparation should be converted into hydrogen ions. Silicates for use as adsorbent in the process according to the invention preferably contain no hydrogen ions. Such silicates can be prepared from the calcined silicates by ion exchange, for instance with an aqueous solution of a sodium salt, followed by calcining. Silicates for use as isomerization catalyst in the process according to the invention preferably have an alkali metal content less than 1 %w and most preferably less than 0.05 %w. Such silicates can be prepared from the above-mentioned calcined silicates by ion exchange, for instance with an aqueous solution of an ammonium salt, followed by calcining.

In the process according to the invention it is preferred to separate, in addition to para-xylene, also ethylbenzene as the end product. As was already explained hereinbefore, pure ethylbenzene may be obtained as the distillate in the distillation of the mother liquor from the crystallization step. The residue obtained in this distillation is preferably recirculated to the crystallization step. In addition to para-xylene, ortho-xylene and meta-xylene can also be separated as end products in the process according to the invention. As was already explained hereinbefore, pure ortho-xylene and meta-xylene can be obtained as the distillates in the distillation of the non-adsorbed product from the first adsorption step. The non-adsorbed product from the second adsorption step is preferably recirculated to the first adsorption step or to the distillation unit in which the non-adsorbed product from the first adsorption step is distilled.

The invention is now explained with the aid of the following example.

Example

Two crystalline silicates which were intended to be used in the process according to the present invention were prepared as follows.

Silicate 1

A mixture of $Fe(NO_3)_3$, $SiO_2$, $NaNO_3$ and $[(C_3H_7)_4N]OH$ in water with the molar composition 0.8 $[(C_3H_7)_4N]_2O$. 0.3 $Na_2O.Fe_2O_3$. 200 $SiO_2$. 55 $H_2O$ was heated for 48 hours in an autoclave at 150°C under autogenous pressure. After the reaction mixture had cooled down, the silicate formed was filtered off, washed with water until the pH of the wash water was about 8 and dried for two hours at 120°C. Silicate A thus prepared had an X-ray powder diffraction pattern substantially as mentioned in Table B of Netherlands patent application No. 7613957. The silicate was thermally stable up to temperatures higher than 900°C and was, after dehydration at 400°C in vacuum, capable of adsorbing 7 %w water at 25°C and saturated water vapour pressure. From silicate A silicate 1 was prepared by, successively, calcining silicate A for four hours at 500°C, boiling with 1.0 molar $NaNO_3$ solution, washing with water, boiling again with 1.0 molar $NaNO_3$ solution and washing, drying for two hours at 120°C and calcining for four hours at 500°C. Silicate 1 thus prepared had the following chemical composition:

$$Na_2O.\ Fe_2O_3.\ 200\ SiO_2.$$

Silicate 2

Silicate B was prepared in substantially the same way as silicate A, the difference being, however, that in the present case the starting material was an aqueous mixture with the molar composition

$$Na_2O.\ 4.5\ [(C_3H_7)_4]_2O.\ Fe_2O_3.\ 29.1\ SiO_2.\ 468\ H_2O$$

Silicate B thus prepared had the following chemical composition:

$$0.67\ [(C_3H_7)_4N]_2O.\ 0.23\ Na_2O.\ Fe_2O_3.\ 30\ SiO_2.\ 9\ H_2O$$

The silicate had an X-ray powder diffraction pattern substantially as mentioned in Table B of Netherlands patent application No. 7613957. The silicate was thermally stable up to temperatures higher than 1000°C and was, after dehydration at 400°C in vacuum, capable of adsorbing 8 %w water at 25°C and saturated water vapour pressure.

From silicate B silicate 2 was prepared in substantially the same way as used in the preparation of silicate 1 from silicate A, the difference being, however, that in the present case an $NH_4NO_3$ solution was used instead of an $NaNO_3$ solution.

Preparation and separation of para-xylene from a mixture of $C_8$ aromatics

A mixture of $C_8$ aromatics comprising ortho-xylene, meta-xylene, para-xylene and ethylbenzene in a weight ratio of 1:2:1:1 was separated by adsorption. To this end the mixture was passed across silicate 1 at 80°C, the silicate was desorbed with toluene and toluene was removed from the desorbate by distillation. In this way two mixtures of $C_8$ aromatics were obtained, of which one mixture consisted of more than 99 %w para-xylene and ethylbenzene, the rest being ortho-xylene and meta-xylene, while the other mixture consisted of more than 99 %w ortho-xylene and meta-xylene, the rest being para-xylene. From the mixture of para-xylene and ethyl-benzene obtained as described above, in which these components were present in a weight ratio of about 1:1, a quantity of para-xylene was separated by cooling down the mixture to a temperature of −60°C. After separation of the crystallized para-xylene from the cooled mixture, a mixture resulted which consisted of more than 99 %w para-xylene and ethylbenzene, the rest being ortho-xylene and meta-xylene. In this mixture para-xylene and ethylbenzene were present in a weight ratio of about 1:6. The mixture of ortho-xylene and meta-xylene obtained as described above, in which these components were present in a weight ratio of about 1:2, was isomerized. To this end the mixture was passed across silicate 2 at a temperature of 250°C and a space velocity of 1 $l.l^{-1}.h^{-1}$. From the isomerizate, which consisted of about 90 %w of a mixture of the three isomeric xylenes, the rest being toluene and $C_9$ aromatics which had been formed in the isomerization, para-xylene was separated by adsorption. To this end the isomerizate, in which ortho-xylene, meta-xylene and para-xylene were present in a weight ratio of about 1:2:1, was passed across silicate 1 at 80°C, the silicate was desorbed with toluene and toluene was removed from the desorbate by distillation. In this way a quantity of para-xylene was obtained and a mixture resulted which consisted of about 95 %w ortho-xylene and meta-xylene, the rest being $C_9$ aromatics. In this mixture ortho-xylene and meta-xylene were present in a weight ratio of about 1:2.

**Claims**

1. A process for the preparation and separation of para-xylene from a mixture substantially consisting of aromatic hydrocarbons with eight carbon atoms in the molecule, in which process the mixture is separated by adsorption at elevated temperature into a mixture of para-xylene and ethylbenzene and a mixture of ortho-xylene and meta-xylene, the para-xylene is separated by crystallization after cooling of the mixture of para-xylene and ethylbenzene, a resulting mixture of ortho-xylene and meta-xylene is isomerized with a catalyst at elevated temperature and a pressure from 1 up to 150 bar, a further quantity of para-xylene is separated from the isomerized product by adsorption at elevated temperature and use is made in both adsorption treatments of a crystalline silicate as adsorbent, characterized in that the crystalline silicate

(a) is thermally stable up to temperatures higher than 600°C,

(b) after dehydration at 400°C in vacuum, is capable of adsorbing more than 3 %w water at 25°C and saturated water vapour pressure, and

(c) in dehydrated form, has the following overall composition, expressed in moles of the oxides:

$$(1.0\pm0.3)(R)_{2/n}O.\ [a\ Fe_2O_3.\ b\ Al_2O_3.\ c\ Ga_2O_3].$$

$y(d\ SiO_2.\ e\ GeO_2)$, where

R=one or more mono- or bivalent cations,

$a\geqslant0.1$,

$b\geqslant0$,

$c\geqslant0$,

$a+b+c=1$,

$y\geqslant10$,

$d\geqslant0.1$,

$e\geqslant0$,

$d+e=1$, and

n=the valency of R.

2. A process according to claim 1, characterized in that a fractional distillation is applied to the feed for the isomerization step with a view to separating part of the ortho- and/or meta-xylene as end product and/or removing undesired components, in particular $C_9^+$ aromatics, from the isomerization feed.

3. A process according to claim 1 or 2, characterized in that the non-adsorbed product from the second adsorption step is recirculated to the isomerization step.

4. A process according to any one of claims 1—3, characterized in that a distillation is applied to the mother liquor of the crystallization step, after para-xylene has been separated from it, with a view to separating ethylbenzene as end product.

5. A process according to claim 4, characterized in that the residue of the distillation carried out for the separation of ethylbenzene as end product, is recirculated to the crystallization step.

6. A process according to any one of claims 1—5, characterized in that the adsorptions are carried out by contacting the feed at a temperature between 25 and 225°C with the adsorbent.

7. A process according to any one of claims 1—6, characterized in that the silicates which are used as the adsorbent do not contain hydrogen ions.

8. A process according to any one of claims 1—6, characterized in that the crystallization is carried out by cooling the feed to a temperature between —15 and —80°C.

9. A process according to any one of the claims 1—8, characterized in that isomerization is carried out in the liquid phase at a temperature lower than 350°C and in the presence of 5—30 %w and preferably of 15—25 %w toluene added as diluent.

10. A process according to claim 9, characterized in that a crystalline silicate of the class of silicates as defined in claim 1 is used as isomerization catalyst.

11. A process according to claim 10, characterized in that the silicates that are used as isomerization catalyst have an alkali metal content of less than 1 %w and preferably of less than 0.05 %w.

12. A process according to any one of claims 1—11, characterized in that crystalline silicates are used of which, in the formula giving the overall composition, a is greater than 0.5 and preferably equal tc 1.

13. A process according to any one of claims 1—12, characterized in that crystalline silicates are used of which, in the formula giving the overall composition, y is less than 600 and preferably less than 300.

**Patentansprüche**

1. Verfahren zur Herstellung und Abtrennung von para-Xylol von einem im wesentlichen aus aromatischen Kohlenwasserstoffen mit 8 Kohlenstoffatomen im Molekül bestehenden Gemisch, in welchem das Gemisch durch Adsorption Bei erhöhter Temperatur in ein Gemisch aus para-Xylol und Äthylbenzol sowie ein Gemisch aus ortho-Xylol und meta-Xylol aufgetrennt wird, das para-Xylol nach Abkühlen des aus para-Xylol und Äthylbenzol bestehenden Gemisches durch Kristallisieren abgetrennt wird, ein dabei enstehendes Gemisch aus ortho-Xylol und meta-Xylol mit Hilfe eines Katalysators bei erhöhter Temperatur und einem Druck von 1 bis 150 bar isomerisiert wird, ein weiterer Anteil des para-Xylols von dem isomerisierten Produkt durch Adsorption bei erhöhter Temperatur abgetrennt wird und in welchem bei beiden Adsorptionsbehandlungen als Adsorbens ein kristallines Silikat verwendet wird, dadurch gekennzeichnet, dass das kristalline Silikat

(a) bis zu Temperaturen über 600°C thermisch stabil ist,

(b) nach der Dehydratisierung bei 400°C im Vakuum die Fähigkeit besitzt, bei einer Temperatur von 25°C und Wassersättingungsdampfdruck über 3 Gewichtsprozent Wasser zu adsorbieren und

(c) in der dehydratisierten Form die folgende Gesamtzusammensetzung, ausgedrückt in Mol der Oxide, aufweist:

$(1,0\pm0,3)\ (R)_{2/n}O.\ [a\ Fe_2O_3.\ b\ Al_2O_3.\ c\ Ga_2O_3].$
$y(d\ SiO_2.\ e\ GeO_2)$, wobei
R=ein oder mehrere ein- oder zweiwertige Kationen bedeutet,
$a\geqslant0,1$
$b\geqslant0$
$c\geqslant0$
$a+b+c=1$
$y\geqslant10$
$d\geqslant0,1$
$e\geqslant0$
$d+e=1$ und
n=der Wertigkeit von R entspricht.

2. Verfahren nach Anspruch 1, dadurch gekennzeichnet, dass das Einsatzmaterial für den Isomerisierungsschritt einer fraktionierten Destillation unterworfen wird, um einen Teil des ortho- und/oder meta-Xylol als Endprodukt abzutrennen und/oder unerwünschte Bestandteile, insbesondere $C_9^+$-Aromaten, aus dem zu isomerisierenden Einsatzmaterial zu entfernen.

3. Verfahren nach Anspruch 1 oder 2, dadurch gekennzeichnet, dass das nicht adsorbierte Produkt aus dem zweiten Adsorptionsschritt in den Isomerisierungsschritt rückgeführt wird.

4. Verfahren nach einem der Ansprüche 1 bis 3, dadurch gekennzeichnet, dass die Mutterlauge aus dem Kristallisierungsschritt destilliert wird, nachdem para-Xylol aus dieser abgetrennt worden ist, um Äthylbenzol als Endprodukt abzutrennen.

5. Verfahren nach Anspruch 4, dadurch gekennzeichnet, dass der bei der Destillation zur Abtrennung von Äthylbenzol als Endprodukt erhaltene Rückstand in den Kristallisierungsschritt rückgeführt wird.

6. Verfahren nach einem der Ansprüche 1 bis 5, dadurch gekennzeichnet, dass die Adsorptionen so durchgeführt werden, dass man das Einsatzmaterial mit dem Adsorbens bei Temperaturen zwischen 25 und 225°C in Berührung bringt.

7. Verfahren nach einem der Ansprüche 1 bis 6, dadurch gekennzeichnet, dass die als Adsorbens verwendeten Silikate keine Wasserstoffionen enthalten.

8. Verfahren nach einem der Ansprüche 1 bis 6, dadurch gekennzeichnet, dass die Kristallisierung so durchgeführt wird, dass man das Einsatzmaterial auf eine Temperatur zwischen —15 und —80°C abkühlt.

9. Verfahren nach einem der Ansprüche 1 bis 8, dadurch gekennzeichnet, dass die Isomerisierung in der Flüssigphase bei einer Temperatur unter 350°C und in Gegenwart von 5 bis 30 Gewichtsprozent, und vorzugsweise von 15 bis 25 Gewichtsprozent, von als Verdünnungsmittel zugesetztem Tuluol durchgeführt wird.

10. Verfahren nach Anspruch 9, dadurch gekennzeichnet, dass als Isomerisierungskatalysator ein wie in Anspruch 1 definiertes kristallines Silikat verwendet wird.

11. Verfahren nach Anspruch 10, dadurch gekennzeichnet, dass die als Isomerisierungskatalysator verwendeten Silikate einen Alkalimetallgehalt von unter 1 Gewichtsprozent, vorzugsweise von unter 0,05 Gewichtsprozent, aufweisen.

12. Verhafren nach einem der Ansprüche 1 bis 11, dadurch gekennzeichnet, dass kristalline Silikate verwendet werden, bei denen in der die Gesamtzusammensetzung wiedergebenden Formel $a>0{,}5$ und vorzugsweise gleich 1 ist.

13. Verfahren nach einem der Ansprüche 1 bis 12, dadurch gekennzeichnet, dass kristalline Silikate verwendet werden, bei denen in der die Gesamtzusammensetzung wiedergebenden Formel y unter 600 und vorzugsweise unter 300 ist.

**Revendications**

1. Procédé de préparation et de séparation de para-xylène à partir d'un mélange constitué essentiellement d'hydrocarbures aromatiques ayant huit atomes de carbone dans la molécule, selon lequel le mélange est séparé par adsorption à température élevée en un mélange de para-xylène et d'éthylbenzène et un mélange d'ortho-xylène et de méta-xylène, le para-xylène est séparé par cristallisation après refroidissement du mélange de para-xylène et d'éthylbenzène, un mélange résultant d'ortho-xylène et de méta-xylène est isomérisé avec un catalyseur à température élevée et à une pression comprise entre 1 et 150 bars, une quantité supplémentaire de para-xylène est séparée à partir du produit isomérisé par adsorption à température élevée et on utilise dans les deux traitements d'adsorption un silicate cristallin comme adsorbant, caractérisé en ce que le silicate cristallin

(a) est thermiquement stable jusqu'à des températures supérieures à 600°C,

(b) après déshydratation à 400°C sous vide, est capable d'adsorber plus de 3% en poids d'eau à 25°C et à la pression de vapeur saturante et

(c) à l'état déshydraté, a la composition d'ensemble suivante, exprimée en moles des oxydes:

$(1{,}0\pm0{,}3)(R)_{2/n}O.\ [a\ Fe_2O_3.\ b\ Al_2O_3.\ c\ Ga_2O_3].\ y(d\ SiO_2.\ e\ GeO_2)$, dans laquelle

R=un ou plusieurs cations mono- ou bivalents,

$a\geqslant0{,}1$

$b\geqslant0$

$c\geqslant0$

$a+b+c=1$

$y\geqslant10$

$d\geqslant0{,}1$

$e\geqslant0$

$d+e=1$ et

n=la valence de R.

2. Procédé selon la revendication 1, caractérisé en ce qu'on applique une distillation fractionnée à la charge pour l'étape d'isomérisation en vue de séparer une partie de l'ortho- et/ou du méta-xylène comme produit final et/ou d'éliminer des constituants indésirables, en particulier des hydrocarbures aromatiques en $C_9^+$, de la charge pour l'isomérisation.

3. Procédé selon la revendication 1 ou 2, caractérisé en ce qu'on recycle à l'étape d'isomérisation le produit non adsorbé dans la deuxième étape d'adsorption.

4. Procédé selon 1'une quelconque des revendications 1 à 3, caractérisé en ce qu'on applique une distillation à la liqueur-mére résultant de l'étape de cristallisation, après que le paraxylène en a été séparé, en vue de séparer de l'ethylbenzène comme produit final.

5. Procédé selon la revendication 4, caractérisé en ce qu'on recycle à l'étape de cristallisation le résidu de la distillation effectuée pour la séparation d'éthylbenzène comme produit final.

6. Procédé selon 1'une quelconque des revendications 1 à 5, caractérisé en ce qu'on effectue les adsorptions en mettant en contact la charge à une température comprise entre 25 et 225°C avec 1'adsorbant.

7. Procédé selon 1'une quelconque des revendications 1 à 6, caractérisé en ce que les silicates qui sont utilisés comme adsorbant ne contiennent pas d'ions hydrogène.

8. Procédé selon 1'une quelconque des revendications 1 à 6, caractérisé en ce qu'on effectue la cristallisation en refroidissant la charge à une température comprise entre —15 et —80°C.

9. Procédé selon 1'une quelconque des revendications 1 à 8, caractérisé en ce qu'on conduit l'isomérisation dans la phase liquide à une température inférieure à 350°C et an présence de 5 à 30% en poids et de préférence de 15 à 25% en poids de toluène adjouté comme diluant.

10. Procédé selon la revendication 9, caractérisé en ce qu'on utilise comme catalyseur d'isomérisation un silicate cristallin de la classe de silicates définie dans la revendication 1.

11. Procédé selon la revendication 10, caractérisé en ce que les silicates qui sont utilisés comme catalyseur d'isomérisation ont une teneur en métaux alcalins de moins de 1% en poids et de préférence moins de 0,05% en poids.

12. Procédé selon 1'une quelconque des revendications 1 à 11, caractérisé en ce qu'on utilise des silicates cristallins pour lesquels, dans la formule donnant la composition d'ensemble a est supérieur à 0,5 et de préférence est égal à 1.

13. Procédé selon 1'une quelconque des revendications 1 à 12, caractérisé en ce qu'on utilise des silicates cristallins pour lesquels, dans la formule donnant la composition d'ensemble, y est inférieur à 600 et de préférence inférieur à 300.